# EUROPEAN PATENT APPLICATION

(11) **EP 1 273 297 A1**
(43) Date of publication of application: **08.01.2003**
(21) Application number: 01116429.0
(22) Date of filing: 06.07.2001
(51) Int. Cl.: A61K 31/355, A61P 31/18, A61P 37/04, A61P 9/10

(54) **Vitamin E for the treatment of diseases in which the binding of the transcription factor NF-KB and/or AP-1 to promoter target sites has a beneficial effect (eg AIDS, cardiac infarction)**

(71) Applicant: Deutsches Krebsforschungszentrum Stiftung des öffentlichen Rechts, 69120 Heidelberg (DE)
(72) Inventor: Krammer, Peter, 69120 Heidelberg (DE); Li-Weber, Min, 69126 Heidelberg (DE)
(74) Representative: Schüssler, Andrea, Dr.

(57) **Abstract**

Described is the use of vitamin E for the preparation of a pharmaceutical composition for the prevention or therapy of a disease in which inhibiting or reducing the binding of the transcription factor NF-KB and/or AP-1 to promoter target sites has a beneficial effect. A preferred use is the treatment of a disease associated with T cell depletion

## Description

The present invention relates to the use of vitamin E for the preparation of a pharmaceutical composition for the prevention or therapy of a disease in which inhibiting or reducing the binding of the transcription factor NF-κB and/or AP-1 to promoter target sites has a beneficial effect. A preferred use is the treatment of a disease associated with T cell depletion.

The acquired immune deficiency syndrome (AIDS) is characterized by a depletion of T cells due to massive apoptosis, at least in part. It has been found that in plasma samples of both HIV-positive individuals and AIDS patients the levels of lipid peroxides are increased. This increase is frequently associated with a decrease in vitamin E levels in serum. Studies in humans and in mouse models have shown that diet supplementation with vitamin E increases CD4⁺ and total lymphocyte counts. However, little is known about the molecular mechanism by which vitamin E enhances T cell numbers. Vitamin E (α-tocopherol), a naturally occurring effective lipid-soluble antioxidant, can prevent toxicant- and carcinogen-induced oxidative damage by trapping reactive oxyradicals. It is a constituent of all cellular membranes and is found in high concentrations in the membranes of immune cells. Vitamin E is essential for normal immune function. Deficiency in Vitamin E has been shown to be associated with increased rates of infection and incidence of tumors. Supplementation of vitamin E in the diet significantly improves the decreased cellular immune function that occurs with aging and, particularly, enhances the immune response in HIV-infected patients. Both studies in a mouse AIDS model and epidemiological statistics support the beneficial effects of vitamin E on preventing infection and decreasing the risk of progression to AIDS. However, so far the molecular basis for vitamin E effects and, thus, its potential use for the prevention and/or therapy of additional diseases, is unknown.

Thus, the technical problem underlying the present invention is to provide uses of vitamin E for a broad spectrum of diseases.

The solution to said technical problem is achieved by providing the embodiments characterized in the claims. The molecular mechanism by which vitamin E downregulates CD95L expression and thereby blocks activation-induced-cell-death (AICD) in T cells could be elucidated: It could be shown by the experiments leading to the present invention that vitamin E affects activities of the transcription factors NF-κB and AP-1 and suppresses expression of the CD95(APO-1/Fas)ligand essential for activation-induced-cell-death (AICD)of T cells. Thus, vitamin E protects T-cells isolated from HIV-infected individuals from CD95-mediated apoptosis. Accordingly, it can be concluded that the application of vitamin E is not only useful for the therapy of AIDS patient but generally for the prevention or therapy of diseases in which inhibiting or reducing the binding of the transcription factor NF-κB and/or AP-1 to promoter target sites has a beneficial effect. Since vitamin E is well tolerated and has few, if any, toxic effects when taken at high doses, it can be applied at very high doses, if required. Moreover, supplementation of vitamin E may also improve the treatment during administration of therapeutic drugs.

Accordingly, the present invention relates to the use of vitamin E for the preparation of a pharmaceutical composition for the prevention or therapy of a disease in which inhibiting or reducing the binding of the transcription factor NF-κB and/or AP-1 to promoter target sites has a beneficial effect, e.g. diseases in which inhibition of CD95-dependent or CD95-independent apoptosis has a therapeutic effect. Examples of such diseases are myocardial infarction, stroke and neurodegenerative diseases.

In a further embodiment, the present invention relates to the use of vitamin E for the preparation of a pharmaceutical composition for the prevention and/or treatment of a disease, wherein the disease is a disease in which inhibiting or reducing the expression of the gene encoding the CD95(APO-1/Fas)ligand has a beneficial effect, e.g. to prevent anti-cancer-drug induced damage of normal tissue, particularly a disease associated with T cell depletion, e.g. AIDS.

In a still further embodiment, the present invention relates to the use of vitamin E for the preparation of a pharmaceutical composition for preventing and/or treating a disease in which inhibiting or reducing CD95-mediated apoptosis has a beneficial effect, preferably said disease is a disease associated with an abnormal immune regulation,e.g AIDS, a disease associated with apoptosis of brain cells, e.g stroke, neurodegenerative diseases, or cardiac infarction.

For administration, vitamin E is preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of vitamin E may be effected by different ways, e.g. by oral, intravenous, intraperetoneal, subcutaneous, intramuscular, topical or intradermal administration. The route of administration, of course, depends on the nature of the disease to be treated. The dosage regimen will be determined by the attending physician and other clinical factors. As is well known in the medical arts, dosages for any one patient depends on many factors, including the patient's size, body surface area, age, sex, time and route of administration, the kind of the disease, general health and other drugs being administered concurrently. Preferred routes of administration are oral, intravenous, intramuscular administration. Preferred dosages are in the range of 300% of the 1989 U.S. Recommended Dietary Allowance or 15-fold higher than the vitamin E level contained in normal diet.

### BRIEF DESCRIPTION OF THE DRAWINGS

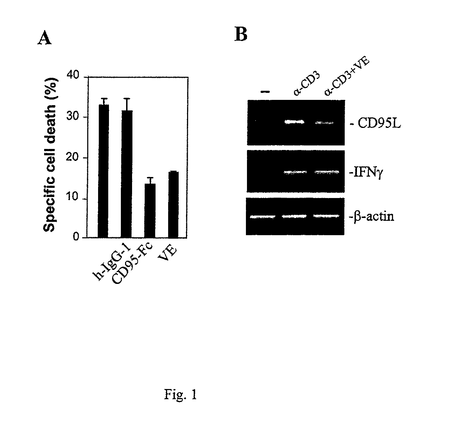

### Fig. 1: Effect of vitamin E on AICD of human peripheral blood T cells

### (A) Vitamin E prevents AICD of peripheral blood T cells

Freshly isolated blood T cells were stimulated by PHA and cultured in the presence of IL-2 for 6 days as described in Klas et al., Int. Immunol. 5 (1993), 625. The day 6 prestimulated T cells were then treated without or with 25 µM vitamin E (VE) Sigma (St. Louis, MO, USA) for 1 h and subsequently cultured in 96-well-flat-bottomed plates coated with α-CD3 (OKT3, 50 µg/ml; (DKFZ, Prof. Krammer) in the absence or presence of CD95-Fc (50 µg/ml; (DKFZ, Prof. Krammer, Dhein et al., Nature 373 (1995), 438) or control human (50 µg/ml) IgG-1. Cell death was assessed after a further 48 h by propidium iodide (PI) uptake (Klas et al., Int. Immunol. 5 (1993), 625.

### (B) Vitamin E suppresses CD95L mRNA levels

The day 6 T cells were cultured in the absence or presence of 25 µM vitamin E for 1 h and then stimulated for 12 h with plate bound α-CD3. The mRNA was analyzed by RT-PCR using primers specific for CD95L (Eichhorst et al., Mol. Cell. Biol. 20 (2000), 7826) . For controls, primers for IFNγ and β-actin were used. The primers had the following sequences:
CD95L: sense-5'-ATAGGATCCATGTTTCTGCTCTTCCACCTACAGAAGGA-3' antisense-5'ATAGAATTCTGACCAAGAGAGGCTCAGATACGTTGAC-3'
IFN-γ: sense-5'AGTTATATCTTGGCTTTTCA-3' antisense-5'ACCGAATAATTAGTCAGCTT-3'
human β-actin: sense-5'TGACGGGGTCACCCACACTGTGCCCATCTA-3' antisense-5'CTAGAATTTGCGGTGGACGATGGAGGG-3'

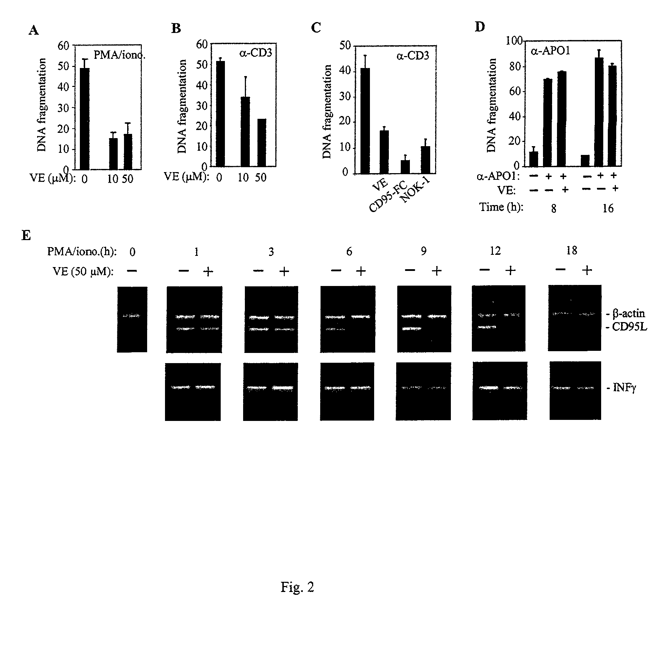

### Fig. 2: Protection of Jurkat J-27 T cells from AICD by vitamin E

### (A) and (B) Vitamin E prevents apoptosis in J-27 T cells

J-27 T cells were induced either with PMA (10 ng/ml) Sigma (St. Louis, MO, USA) and ionomycin (0.5 µM), Calbiochem (Bad Soden/Ts, Germany) or with plate bound α-CD3 (OKT3, 50 µg/ml) for 24 h in the presence of vitamin E or equal amount of the vitamin E solvent ethanol. Apoptotic nuclei were measured by determination of DNA fragmentation (Dhein et al., Nature 373 (1995), 438).

### The CD95L blocker, CD95-Fc and NOKI, blocked AICD

J-27 cells were stimulated in the absence or presence of CD95-Fc (50 µg/ml) or NOKI (50 µg/ml). BD-Pharmingen (Heidelberg, Germany), for 24 h.

### (D) Vitamin E does not block α-APO-1 induced apoptosis

J-27 cells were cultured in the absence or presence of 50 µM vitamin E for 1 h and than, treated with α-APO-1 (10 ng/ml) antibody, DKFZ, Prof. Krammer, Trauth et al., Science 245 (1989), 301 in the presence of 10 ng/ml protein A for the indicated times.

### (E) Vitamin E downregulated CD95L mRNA expression in J-27 cells

J-27 T cells were cultured in the absence or presence of 50 µM vitamin E for 1 h and then stimulated by PMA (10 ng/ml) and ionomycin (0.5 µM) for the indicated times. CD95L mRNA expression was analyzed by RT-PCR. Primers for IFNγ and β-actin were used as controls.

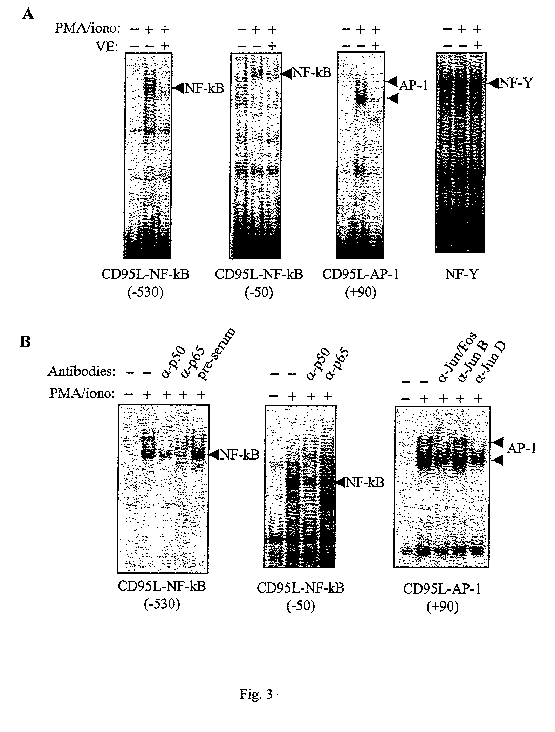

### Fig. 3: Vitamin E blocks binding of NF-κB and AP-1 to the CD95L promoter target sites

**(A)**³²P-labeled oligonucleotides containing the CD95L NF-κB (at the promoter -530 and -50 region) (Li-Weber et al., Eur. J. Immunol. 30 (2000), 661) and AP-1 (at the promoter +90 region) (Eichhorst et al., Mol. Cell. Biol. 20 (2000), 7826) binding sequences were analyzed by EMSA as described (Li-Weber et al., (2000). The EMSA probes had the following sequences:
CD95L NF-κB at-530: 5'-GCCTCCATGGTCTCTCCCCTCAGAGCCATT-3'
CD95L NF-κB at-50: 5'-AGACAGAGGTGTTTCCCTTAGCTATGGA-3'
CD95L AP-1 at +90: 5'-GGGCTGGCCTGACTCACCAGCTGC-3'

Nuclear extracts were prepared from J-27 T cells either non-induced (-) or induced (+) for 2 h by PMA (10 ng/ml) and ionomycin (0.5 µM) in the absence (-) or presence (+) of 50 µM vitamin E (VE) (added 1 h before induction). The inducible complex corresponding to NF-κB and AP-1 were indicated. A NF-Y binding oligonucleotide 5'CCACTTTTAACCAATCAGAAAAAT-3' was used for equal loading of nuclear extracts.

### (B) EMSA was carried out in the absence (-) or presence of antibodies (1 to 2 µl) indicated.

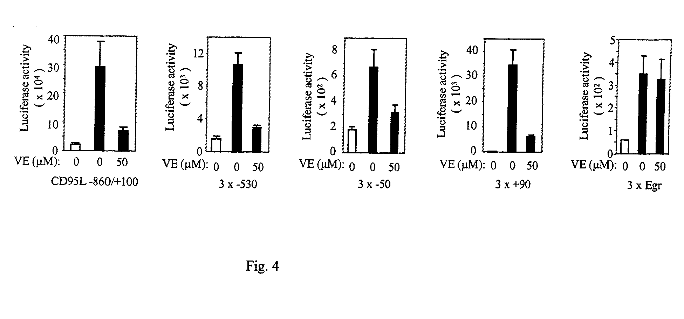

### Fig. 4: Vitamin E downregulates the CD95L promoter and CD95L NF-κB- or CD95L AP-1-mediated transcriptional activity

J-27 T cells were transiently transfected with the luciferase reporter constructs containing the 0.86-kb CD95L promoter (Li-Weber et al., Eur. J. Immunol. 30 (2000), 661), or three copies of the NF-κB binding sites at -530 or -50 region (Li-Weber et al., Eur. J. Immunol. 30 (2000), 661) or the AP-1 motif at +90 region (Eichhorst et al., Mol. Cell. Biol. 20 (2000), 7826) of the CD95L promoter as described in Li-Weber et al., 2000. All promoter constructs are described in the cited references. A luciferase reporter construct containing three copies of the Egr consensus binding site was used as a control (Li-Weber et al., Eur. J. Immunol. 29 (1999), 3017). After overnight culture, the cells were divided and treated with vitamin E (VE) (50 µM) or the vitamin E solvent ethanol for 1 h and than further cultured in the absence (empty box) or presence (black box) of PMA (10 ng/ml) and ionomycin (0.5 µM). Luciferase activity was determined after 8 h of stimulation. Results represent the average of three to five independent transfection experiments.

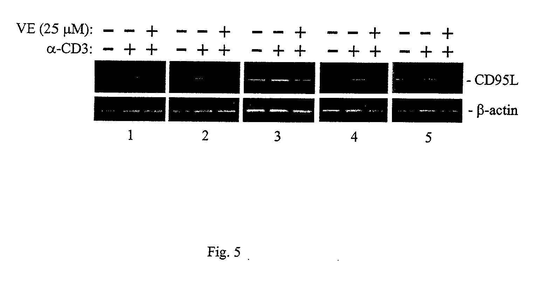

### Fig. 5: Effect of vitamin E on CD95L mRNA levels in peripheral blood lymphocytes of HIV positive individuals

Freshly isolated peripheral blood lymphocytes from HIV positive individuals were treated without or with vitamin E (VE) (25 µM) for 1 h and than stimulated with plate bound α-CD3 (50 µ/ml) for 12 h. CD95L mRNA levels were examined by RT-PCR (Eichhorst et al., 2000). Results from 5 individuals are shown.

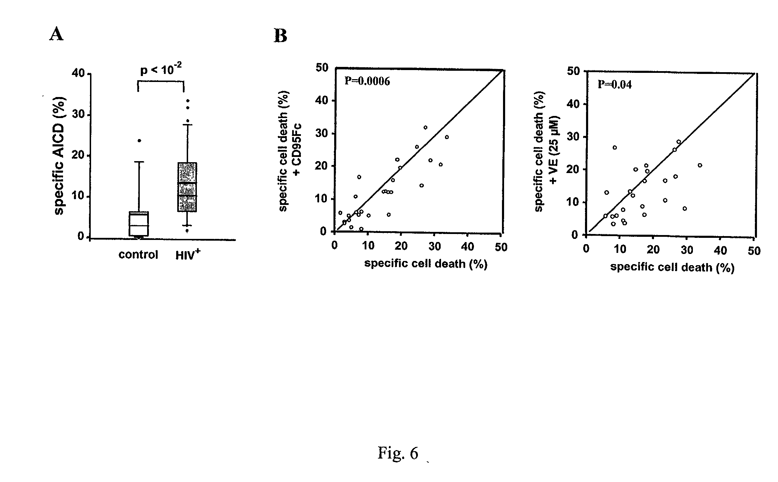

### Fig. 6. Effect of vitamin E on AICD of peripheral blood lymphocvtes of HIV positive individuals

### (A) AICD is increased in HIV infected individuals

Peripheral blood lymphocytes from HIV positive individuals (n = 33 Patients) and uninfected donors (11 Donors) were stimulated with plate bound (α-CD3 (50 µg/ml) for 48 h. Apoptosis was assessed by a drop in the forward to side scatter profile of apoptotic in comparison to living cells. The mean difference between patients and uninfected individuals is -7.23. 95% confidence interval is -13.7, -2.12.

### (B) Vitamin E prevents AICD of Iymphocytes of HIV positive individuals

Freshly isolated blood lymphocytes from 31 HIV positive individuals were stimulated with plate bound α-CD3 in the absence or presence of either 50 µg/ml CD95-Fc or 25 µM vitamin E (VE) (added 1 h before stimulation). IgG-1 (50 µg/ml), Sigma (St. Louis, MO, USA) was used as control. Cell death was assessed after 48 h stimulation.

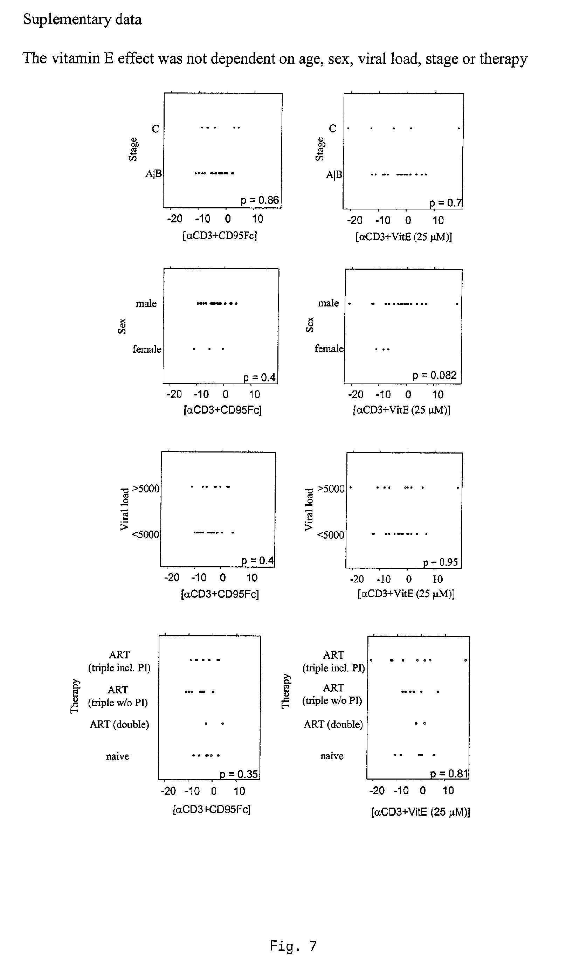

### Figure 7: Effect of vitamin E is not dependent on age, sex, viral load, stage or therapy

To investigate whether other factors influence the vitamin E efficiency we analyzed the vitamin E effect and the histroy of patients by statistic evaluation. The analysis showed that there are no correlation between the vitamin E effect and age, sex, viral load and stage or therapy of patients.

The following Examples illustrate the invention.

### EXAMPLE 1: Studies on the effect of vitamin E on AICD of human peripheral blood cells

Since activation-induced-cell-death (AICD) is a major cause of T cell depletion in AIDS and since supplementation of vitamin E is shown to increase T cell numbers, it was investigated whether vitamin E could protect from T cell depletion by AICD. To analyze the effect of vitamin E on T cell death, freshly isolated peripheral blood T cells were prestimulated for 6 days (referred to as day 6 cells) and subsequently restimulated via the TCR by plate-bound α-CD3 antibodies. Restimulation of the day 6 T cells resulted in AICD (Fig. IA). CD95 (APO-1/Fas) and its ligand (CD95L) are known to play a major role in AICD of T cells. As shown in Fig. 1A, in the presence of the CD95 blocker, CD95-Fc, AICD was inhibited. A similar inhibition of AICD was obtained in the presence of 25 µM vitamin E in the culture medium (Fig. 1A). The concentration of vitamin E in plasma from healthy individuals normally ranges from 12-35 µM. Thus, vitamin E at physiological concentrations may attenuate AICD. Indeed, depending on the blood donors, vitamin E reduced AICD by 20-50%. Since CD95-mediated AICD is regulated by CD95L expression, CD95L mRNA expression levels in activated T cells were examined in the presence or the absence of vitamin E. Restimulation of day 6 T cells led to enhanced expression of CD95L mRNA. This increase was blocked in the presence of vitamin E (Fig.1B). In contrast, vitamin E did not significantly influence the levels of IFNγ mRNA (Fig. 1B). Thus, downregulation of CD95L expression may account for the reduction of AICD by vitamin E. Experimental details are given in the legend to Figure 1.

### Example 2: Protection of Jurkat J-27 cells from AICD by vitamin E

To further investigate the molecular mechanism by which vitamin E attenuates AICD, several Jurkat T cell sub-clones susceptible to T-cell-receptor (TCR)-mediated-activation-induced apoptosis were selected. Data from one of the representative clones, clone 27 (J-27) is presented here. Activation of T cells through the T cell receptor can be mimicked by stimulation with the phorbol ester (PMA) and calcium ionophores (ionomycin). Both α-CD3 or PMA/ionomycin-induced cell death in J-27 cells were significantly reduced by vitamin E (Fig. 2A, B). As controls, the CD95 blocker, CD95-Fc, or the CD95L blocking antibody NOKI greatly inhibited AICD (Fig. 2C). However, vitamin E did not prevent monoclonal antibody α-APO-1 induced CD95-triggered apoptosis (Fig. 2D). Therefore, it is unlikely that vitamin E directly interferes with signals downstream of the CD95 receptor. Analysis of CD95L mRNA expression showed that, upon TCR activation, CD95L mRNA in J-27 cells was rapidly induced and its expression increased for up to 12 h. In the presence of vitamin E, however, CD95L mRNA expression was substantially reduced. The reduction was readily seen at 3 h and was nearly completely blocked by vitamin E 6 h post stimulation (Fig. 2E). In contrast, the levels of IFNγ mRNA were not significantly changed. Thus, vitamin E may reduce CD95L expression at the transcriptional level and, therefore, attenuate AICD in J-27 cells. Experimental details are given in the legend to Figure 2.

### Example 3: Vitamin E blocks binding of NF-κB and AP-1 to the CD95L promoter target sites

It was investigated whether vitamin E affects NF-KB and AP-1 binding to the CD95L promoter target sites. Nuclear extracts were prepared from J-27 T cells stimulated in the presence or absence of vitamin E. The NF-κB and AP-1 binding sites contained in the CD95L promoter were used as probes in an electrophoretic-mobility-shift-assay (EMSA). Stimulation of T cell with PMA and ionomycin induced binding of nuclear factors to the NF-κB and AP-1 sites of the CD95L promoter (Fig. 3A). These inducible binding complexes were either reduced or supershifted in the presence of antibodies against the subunits p50 and p65 of NF-κB or antibodies against the AP-1 family members Jun and Fos (Fig. 3B). Addition of vitamin E prevented binding of NF-κB and AP-1 to their binding sites (Fig. 3A). As a control, the constitutive transcription factor NF-κB was not affected by vitamin E (Fig. 3A). Experimental details are given in the legend to Figure 3.

### Example 4: Vitamin E downregulates the CD95L promoter and CD95L NF-κB or CD95L AP-1-mediated transcriptional activity

To further investigate the effect of vitamin E on CD95L expression, the CD95L promoter (-860 to +100) or a minimal TATA promoter containing three copies of either the NF-B or the AP1 sites of the CD95L promoter were linked to the luciferase reporter gene and these constructs were transiently transfected into J-27 T cells. After transfection, the cells were stimulated in the absence or presence of vitamin E. In accordance with the above data, vitamin E greatly reduced luciferase expression driven by the CD95L promoter or by the NF-κB and the AP-1 site of the CD95L promoter (Fig. 4). In control, vitamin E did not affect luciferase expression driven by the Egr binding site (Fig. 4). Experimental details are given in the legend to Figure 4.

### Example 5: Studies on the effect of vitamin E on CD95 mRNA levels in peripheral blood lymphocytes of HIV positive individuals

It was investigated whether vitamin E could block CD95L expression in the peripheral blood lymphocytes of HIV positive individuals. Peripheral blood lymphocytes from such individuals were subjected to analysis of the CD95L mRNA expression levels in the absence or the presence of vitamin E. Different levels of the CD95L mRNA expression were found in lymphocytes from different HIV patients examined. In some patients, like in healthy individuals, no or very little CD95L mRNA was expressed in unstimulated cells and CD95L transcripts were significantly upregulated upon α-CD3 stimulation (Fig. 5, lanes 1 and 2).In many patients, however, CD95L mRNA expression was readily detectable in unstimulated cells and was further increased upon TCR-engagement in most cases (Fig. 5, lanes 3 and 4). Nevertheless, in the presence of 25 µM vitamin E, expression of CD95L mRNA was completely blocked in most patients (Fig. 5). Experimental details are given in the legend to Figure 5.

### Example 6: Studies on the effect of vitamin E on AICD of peripheral blood lymphocytes of HIV positive individuals

To determine if vitamin E could reduce depletion of T cells from HIV positive individuals next the effects of vitamin E on AICD in peripheral blood lymphocytes of such individuals were analyzed. Table 1 shows the medical history and the mean values of total lymphocytes, CD8⁺, and CD4⁺ T cells in these patients. As opposed to lymphocytes from healthy donors lymphocytes isolated from HIV-infected individuals were more susceptible to apoptosis (Fig. 6A). Apoptosis of HIV⁺ T cells could partially be blocked by CD95-Fc demonstrating that a fraction of T cells underwent CD95/CD95L-mediated apoptosis (Fig. 6B). In the presence of vitamin E, AICD was greatly reduced (Fig. 6B). The reduction of AICD by vitamin E was even more pronounced than that obtained by CD95-Fc suggesting that vitamin E may also block CD95-independent apoptosis. Statistical analysis showed that the vitamin E effect was not dependent on age, sex, viral load, stage or therapy (Fig. 7). Experimental details are given in the legend to Figure 6.

**Table 1:**

| **Characteristics of patients** | | |
|---|---|---|
| | **Healthy donors**^{**1**} | **HIV**^{**+**} **donors** |
| No. of patients | 11 | 35 |
| Age (years)² | 26 ± 6 | 42 ± 12 |
| Sex (F/M) | 6/5 | 5/30 |
| viral load (log copies/ml)^{2,}³ | ND | 4.24 ± 4.26 |
| CD4⁺ cells (cells/µl)² | ND | 446 ± 287 |
| CD8⁺ cells (cells/µl)² | ND | 1221 ± 544 |
| CD4⁺/CD8⁺ ratio² | ND⁴ | ± 0.25 |
| Lymphocytes (cells/µl)² | ND⁴ | 2041 ± 910 |
| | | |
| | | Native (n=6) |
| Treatment⁵ | - | ART,double (n=4) ART, triple (n=15) HAART, triple + PI (n=10) |
| | | A1 (n=1) |
| Stage (CDC 1993)⁶ | - | B1/B2/B3 (n=1/25/1) C3 (n=7) |

| | | |
|---|---|---|
| ¹p24 negative | | |
| ²numbers are mean ± SD | | |
| ³in n=16 patients viral load was below 50 copies/ml (detection limit), bDNA test (Chiron, Emeryville, Ca., USA). | | |
| ⁴In general, total lymphocytes of healthy individuals are 2182 ± 605, CD4⁺/CD8⁺ ratio is 1.55 ± 0.49. | | |
| ⁵ART: antiretroviral therapy; double, triple: combinations of two or three nucleoside or non-nucleoside analogues; HAART: highly active ART; PI: protease inhibitor. | | |
| ⁶CDC: centers of disease control, classification of 1993. Patients and healthy donors were recruited during Sept. - Dec. 2000. Approval was given by the local ethics committee. | | |

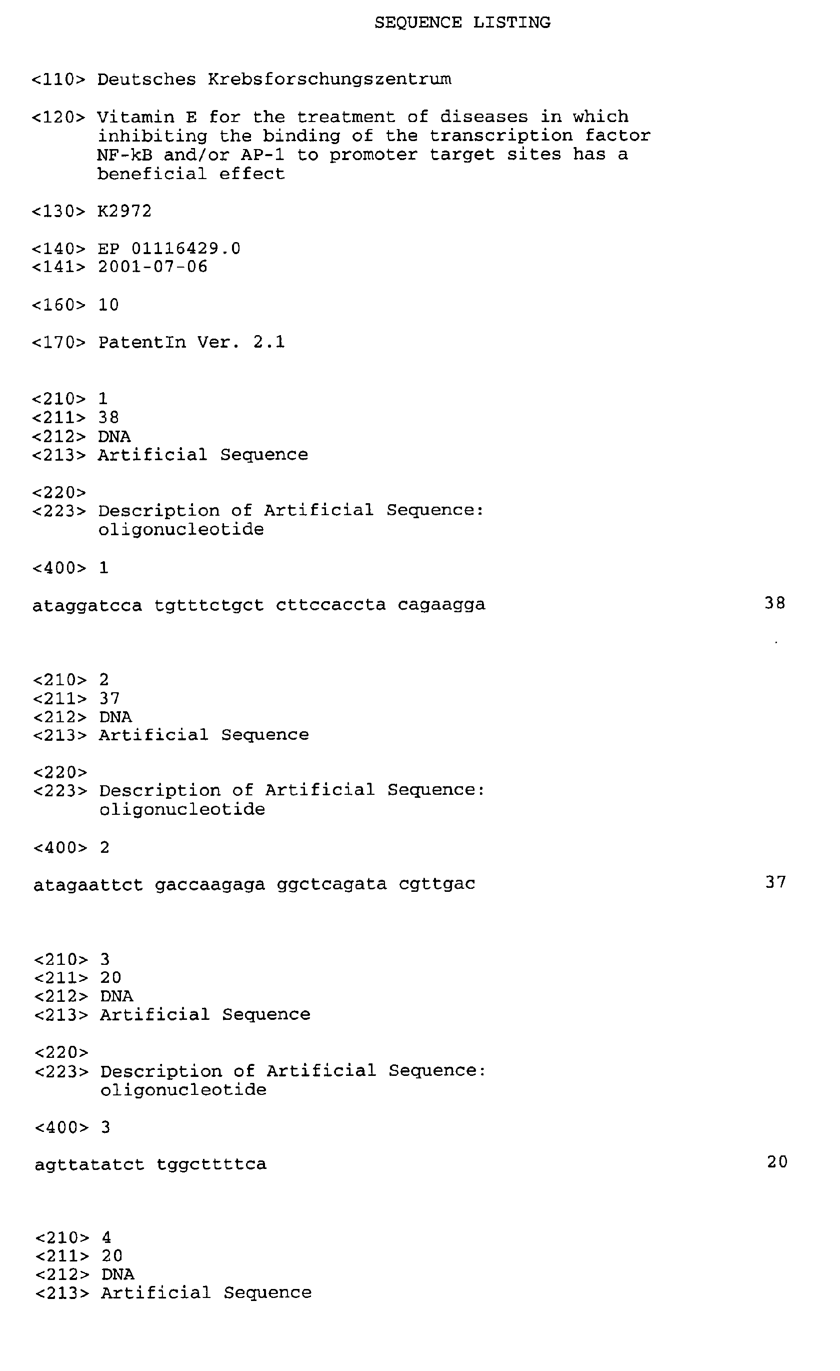

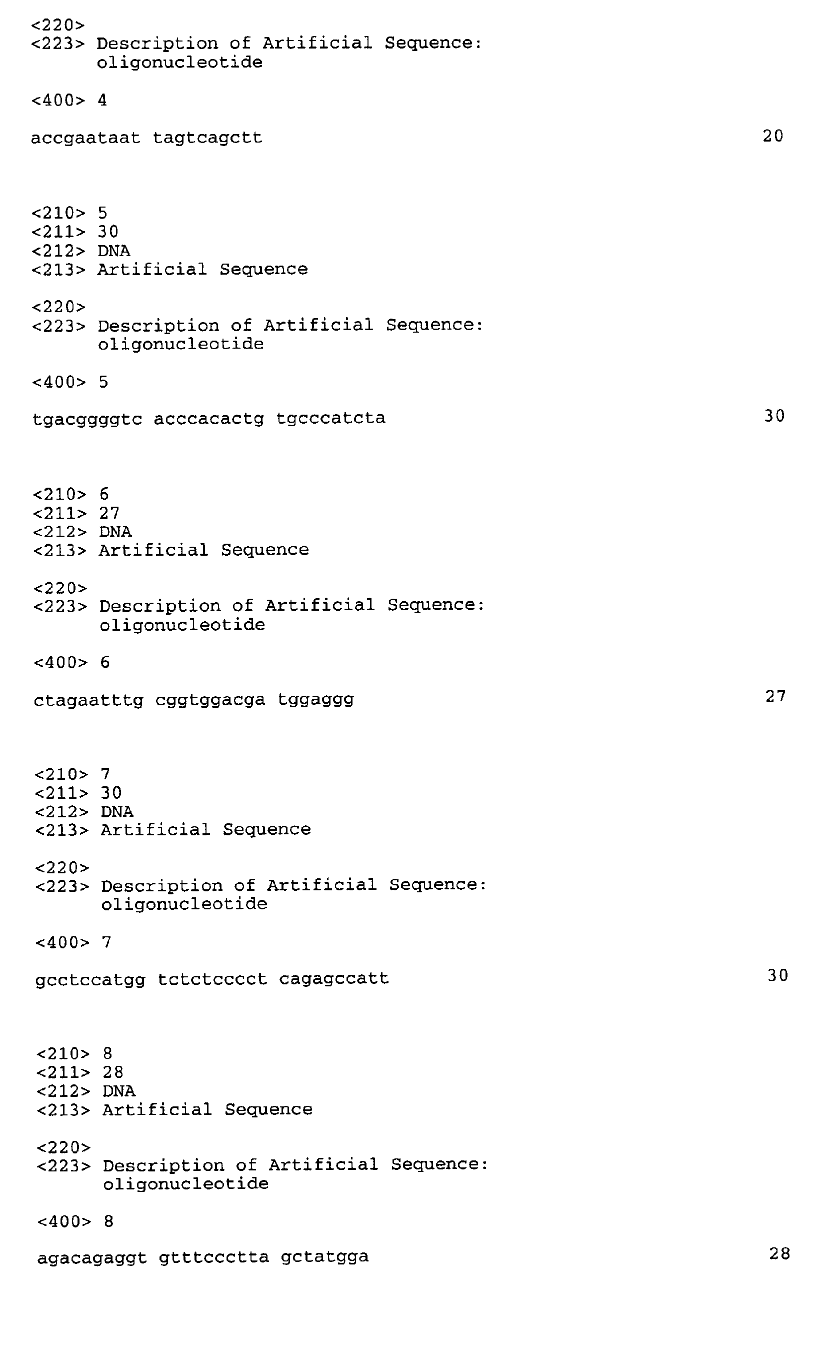

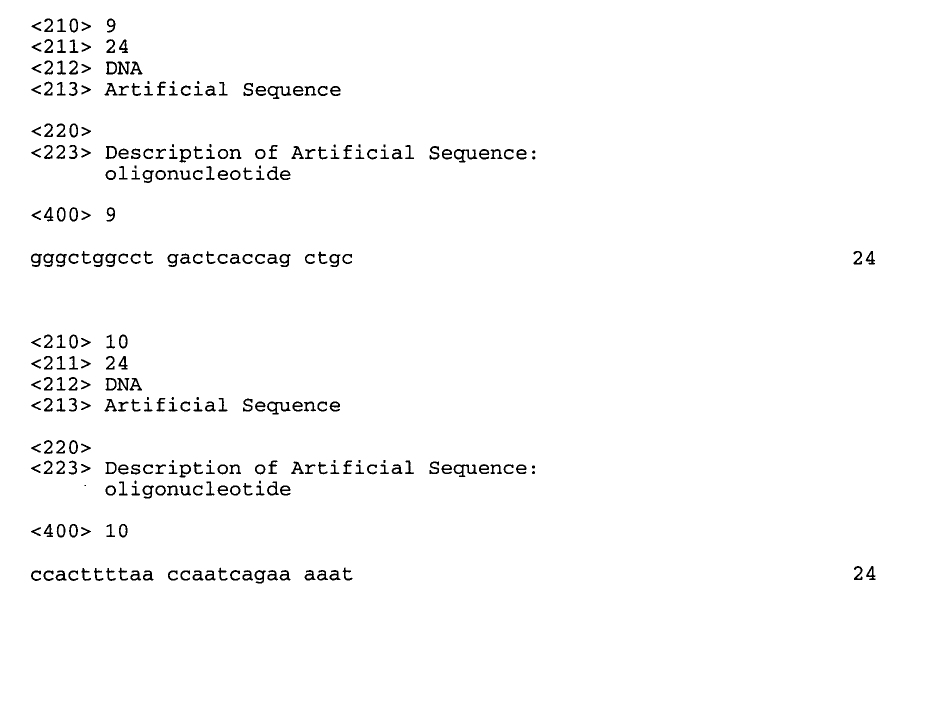

## Claims

1. Use of vitamin E for the preparation of a pharmaceutical composition for the prevention or therapy of a disease in which inhibiting or reducing the binding of the transcription factor NF-κB and/or AP-1 to promoter target sites has a beneficial effect.

2. Use according to claim 1, wherein the disease is a disease in which inhibiting or reducing the expression of the gene encoding the CD95(APO-1/Fas)ligand has a beneficial effect.

3. Use according to claim 2, wherein the disease is a disease associated with T cell depletion.

4. Use according to claim 3, wherein the disease is AIDS.

5. Use according to claim 1 or 2, wherein the disease is a disease in which reducing or inhibiting CD95-mediated apoptosis has a beneficial effect.

6. Use according to claim 5, wherein the disease is a disease associated with an abnormal immune regulation, a disease associated with apoptosis of brain cells or cardiac infarction.
